# DEMANDE DE BREVET EUROPEEN

(11) **EP 3 556 423 A1**
(43) Date de publication de la demande: **23.10.2019**
(21) Numéro de dépôt: 19169959.4
(22) Date de dépôt: 17.04.2019
(51) Int. Cl.: A61M 37/00

(54) **DISPOSITIF D'INJECTION D'IMPLANT DOTÉ D'UNE TRANSMISSION DE TYPE PIGNON-CRÉMAILLÈRE**

(30) Priorité: 18.04.2018 FR 1853406
(71) Demandeur: NEMERA LA VERPILLIERE, 38290 La Verpillière (FR)
(72) Inventeur: PAINCHAUD, Gaëtan, 69340 FRANCHEVILLE (FR); DUGAND, Pascal, 38780 ESTRABLIN (FR); MEGARD, Thomas, 71960 LA ROCHE-VINEUSE (FR)
(74) Mandataire: LLR

(57) **Abrégé**

L'invention concerne un dispositif d'injection d'implant (1), comportant :
- une aiguille d'injection (9),
- un corps de réception (11) d'au moins un implant,
- des moyens d'injection (5), les moyens d'injection (5) comprenant :
• une tige de poussée, disposée en amont de l'implant logé dans le corps de réception (11), s'étendant longitudinalement et configurée pour pousser l'implant au travers de l'aiguille d'injection (9) entre une position initiale et une position finale dans laquelle l'implant est injecté,
• des moyens d'actionnement (33) par un utilisateur, configurés pour actionner un déplacement de la tige de poussée de la position initiale vers la position finale,

les moyens d'actionnement (33) comprenant un mécanisme de transmission à crémaillère (35).

## Description

L'invention concerne le domaine technique de l'injection d'un ou plusieurs implants dans le corps d'un patient.

On connaît des dispositifs d'injection d'implant, comprenant une aiguille creuse fixée à un logement recevant un implant. L'implant est injecté au moyen d'une tige de poussée, laquelle vient pousser l'implant à travers l'aiguille creuse puis au-delà pour permettre l'injection de l'implant dans le corps d'un patient.

On connaît en particulier du document US20090281520A1 un dispositif d'injection d'implant dans lequel l'injection de l'implant peut se faire par appui coulissant sur un bouton, le bouton étant alors entraîné par l'utilisateur dans une direction sensiblement parallèle à la direction d'injection. Ainsi, avant actionnement, la tige de poussée est rétractée et le bouton se trouve du côté de l'extrémité proximale du dispositif, c'est-à-dire l'extrémité opposée à l'extrémité d'injection. Lors de l'appui glissant sur le bouton par l'utilisateur, le bouton coulisse dans une direction sensiblement parallèle à la direction d'injection et entraîne la tige de poussée, laquelle pousse l'implant et permet son injection.

Toutefois, un tel actionnement par appui coulissant ne permet pas aisément à l'utilisateur d'injecter plusieurs implants. En effet, cet actionnement est généralement réalisé par le pouce de l'utilisateur, lequel possède une course réduite pour l'actionnement qui ne suffit pas à la course nécessaire pour l'injection d'un implant de grande longueur et/ou pour l'injection successive de deux implants ou plus. De plus un tel actionnement ne permet pas de maintenir une grande précision d'injection sur une grande course, et ne permet pas en particulier de stopper l'injection lorsque l'injection d'un implant est effectuée, par exemple pour orienter l'aiguille d'injection dans une autre direction pour un second implant afin de ne pas injecter sur une trop longue profondeur. En effet, le risque de débuter de manière non souhaitée l'injection d'un implant suivant l'implant déjà injecté existe avec un tel actionnement. Cela n'est pas souhaitable pour garantir une injection correcte, notamment pour éviter une rupture d'au moins un des implants ou une blessure du patient.

Ainsi, compte tenu de leur type d'actionnement, ces dispositifs d'injection d'implant ne permettent pas d'injecter aisément un implant de grande longueur ou bien une pluralité d'implants.

La présente invention vise notamment à fournir un dispositif d'injection d'implant, lequel permet d'injecter aisément un implant de grande longueur et/ou une pluralité d'implants.

A cet effet, l'invention a notamment pour objet un dispositif d'injection d'implant, comportant :
- une aiguille d'injection,
- un corps de réception d'au moins un implant,
- des moyens d'injection, les moyens d'injection comprenant :
   - une tige de poussée, disposée en amont de l'implant logé dans le corps de réception, s'étendant longitudinalement et configurée pour pousser l'implant au travers de l'aiguille d'injection entre une position initiale et une position finale dans laquelle l'implant est injecté,
   - des moyens d'actionnement par un utilisateur, configurés pour actionner un déplacement de la tige de poussée de la position initiale vers la position finale,
les moyens d'actionnement comprenant un mécanisme de transmission à crémaillère.

Ainsi, on propose de réaliser un injecteur d'implant aisé à manipuler et à assembler, en réalisant une transmission de mouvement agréable pour un utilisateur. En effet, par un ou plusieurs mouvements de faible amplitude, l'utilisateur peut actionner la tige de poussée sur une grande longueur grâce à la transmission à crémaillère, permettant par exemple d'injecter un implant de grande longueur ou une pluralité d'implants.

On comprend qu'un mécanisme de transmission à crémaillère comprend de préférence un élément rectiligne denté coopérant avec une roue dentée, pour transformer un mouvement de rotation en un mouvement de translation ou réciproquement.

On entend de préférence par "implant", un composé pharmaceutique à l'état solide ou semi-solide, par exemple sous forme de liquide encapsulé, et/ou un composant électronique, par exemple une puce électronique pouvant être de type RFID. On entend généralement par "patient" ou "sujet" un être vivant comme par exemple un mammifère, notamment un être humain. L'utilisateur est en général une personne différente du patient, mais il est possible que l'utilisateur soit le patient lui-même.

Dans la présente description, on comprend que la direction distale désigne la direction la plus éloignée des doigts d'un utilisateur, c'est-à-dire la plus proche de la peau ou de la surface d'un patient au moment d'une injection, et la direction proximale désigne la direction opposée à la direction distale. En d'autres termes, on considère que la direction distale et le sens distal sont la direction et le sens qui vont vers « l'avant » du dispositif d'injection d'implant, direction autrement appelée direction d'injection. En particulier, l'extrémité distale d'une pièce correspond à l'extrémité se trouvant du côté de l'aiguille d'injection, et l'extrémité proximale correspond à l'extrémité opposée. On comprend par ailleurs que l'axe d'injection, portant la direction d'injection, correspond à l'axe du dispositif d'injection d'implant, défini par l'axe de l'aiguille d'injection.

On comprend par conséquent que la direction « aval » est une direction opposée à la direction « amont » et correspond à la direction orientée dans le sens de l'extrémité distale du dispositif d'injection d'implant, c'est-à-dire en direction du site d'injection, vers l'extrémité configurée pour se trouver en contact avec le site d'injection de l'implant. Ainsi, la direction « aval » peut également être appelée direction d'injection.

On comprend que les termes « amont » et « aval » sont relatifs aux directions distale et proximale, un élément aval étant disposé plus loin dans la direction distale qu'un élément amont. Le dispositif d'injection d'implant peut en outre comporter l'une ou plusieurs des caractéristiques suivantes, prises seules ou en combinaison.

- Le mécanisme de transmission à crémaillère comporte un premier élément de crémaillère et un deuxième élément de crémaillère,
- le premier élément de crémaillère étant configuré pour être entraîné par l'utilisateur et formant une première liaison de type pignon-crémaillère avec un pignon, le pignon comprenant une première denture coopérant avec le premier élément de crémaillère,
- le pignon comprenant une deuxième denture coopérant avec le deuxième élément de crémaillère configuré pour pousser la tige de poussée de la position initiale vers la position finale.

Ainsi, on propose un mécanisme de transmission à crémaillère comprenant un pignon et deux éléments de crémaillère, de façon à permettre une démultiplication du mouvement de l'utilisateur. En d'autres termes, par un mouvement coulissant sur une faible longueur, entraînant le premier élément de crémaillère, l'utilisateur peut actionner la tige de poussée sur une grande longueur, permettant par exemple d'injecter un implant de grande longueur ou une pluralité d'implants. Avantageusement, la première denture et la deuxième denture du pignon sont réalisées par une première roue dentée et une deuxième roue dentée du pignon, la première roue dentée ayant un diamètre inférieur au diamètre de la deuxième roue dentée.
- Les moyens d'actionnement comportent un bouton d'actionnement coulissant dans une direction sensiblement parallèle à la direction longitudinale de la tige de poussée, lequel est supporté par le premier élément de crémaillère. Ainsi, le mouvement à réaliser pour l'actionnement du dispositif d'injection d'implant est particulièrement aisé pour un utilisateur, lequel peut par exemple facilement maintenir le dispositif d'injection d'implant d'une seule main et actionner la tige de poussée au moyen du bouton d'actionnement coulissant, à l'aide du pouce de cette main.
- Le dispositif d'injection d'implant comporte des moyens d'indication d'une position de la tige de poussée à l'utilisateur. Ainsi, l'utilisateur contrôle facilement la position de la tige de poussée et peut par exemple stopper l'actionnement de la tige de poussée en se basant sur l'indication fournie par les moyens d'indication, ce qui est particulièrement avantageux si l'on indique une position correspondant à la fin d'injection d'un premier implant. En effet, cela permet de prévenir l'utilisateur qu'il peut retirer le dispositif d'injection d'implant ou bien changer d'orientation d'injection pour injecter un deuxième implant à une même profondeur que le premier implant.
- Les moyens d'indication comprennent une première languette flexible portée par un boîtier, et le mécanisme de transmission à crémaillère comprenant au moins une saillie, de telle manière que lorsque le mécanisme de transmission à crémaillère atteint une première position prédéterminée, la saillie se trouve en butée contre la première languette flexible de façon à fournir à l'utilisateur une première indication sonore et/ou tactile. Ainsi, lorsque le mécanisme de transmission à crémaillère atteint une première position prédéterminée, l'utilisateur détecte à l'aide de l'indication sonore et/ou tactile, particulièrement pratique, que le mécanisme de transmission à crémaillère a atteint cette première position prédéterminée, laquelle peut par exemple correspondre à une position dans laquelle un implant est injecté. Cela permet par exemple d'indiquer à l'utilisateur de stopper l'injection lorsque l'injection d'un implant est effectuée, par exemple afin de ne pas injecter sur une trop longue profondeur ou pour orienter l'aiguille d'injection dans une autre direction pour un autre implant ultérieur comme un second implant, afin de ne pas injecter sur une trop longue profondeur.
- Les moyens d'indication comportent une seconde languette flexible portée par le boîtier, la seconde languette flexible étant configurée pour venir en butée contre la saillie dans une seconde position prédéterminée précédant la première position prédéterminée, de telle manière que lorsque le mécanisme de transmission à crémaillère atteint une seconde position prédéterminée, la saillie se trouve en butée contre la seconde languette flexible, et lorsque le mécanisme de transmission à crémaillère dépasse la seconde position prédéterminée, la seconde languette flexible franchit la saillie de façon à fournir à l'utilisateur une seconde indication sonore et/ou tactile. Ainsi, lorsque le mécanisme de transmission à crémaillère atteint une seconde position prédéterminée, laquelle correspond à une position précédant la première position prédéterminée, l'utilisateur détecte à l'aide de l'indication sonore et/ou tactile que le mécanisme de transmission à crémaillère a atteint cette seconde position prédéterminée, laquelle peut par exemple correspondre à une position précédant une position dans laquelle un implant est injecté. Cela permet par exemple d'indiquer à l'utilisateur de ralentir l'injection lorsque l'injection d'un implant est proche d'être effectuée complètement, afin de ne pas injecter sur une trop longue profondeur.
- La première languette flexible comporte une résistance à la flexion plus élevée que celle de la seconde languette flexible. Ainsi, l'indication tactile et/ou sonore est différente selon que le mécanisme de transmission à crémaillère a atteint la seconde position prédéterminée ou la première position prédéterminée. La seconde position prédéterminée peut correspondre à une position intermédiaire d'actionnement dans laquelle une partie d'implant est encore à injecter, et la première position prédéterminée peut correspondre à une position d'actionnement dans laquelle un implant est injecté. Il est alors utile que la première languette flexible comporte une résistance à la flexion plus élevée que la seconde languette flexible, cela permettant à l'utilisateur de détecter facilement que l'implant est injecté, afin de ne pas injecter sur une trop grande profondeur, ou bien pour orienter l'aiguille d'injection dans une autre direction pour un autre implant ultérieur comme un second implant, afin de ne pas injecter sur une trop longue profondeur.
- Le corps de réception est apte à recevoir une pluralité d'implants, la tige de poussée, dans la première position prédéterminée du mécanisme de transmission à crémaillère, occupe une position dans laquelle un implant est injecté. Ainsi, l'utilisateur détecte facilement que l'implant est injecté, afin de ne pas injecter sur une trop grande profondeur, ou bien pour orienter l'aiguille d'injection dans une autre direction pour un autre implant afin de ne pas injecter sur une trop longue profondeur.
- La au moins une saillie comprend une dent protubérante d'une surface longitudinale plane du mécanisme de transmission à crémaillère. Ainsi, les moyens d'indications sont réalisés de manière particulièrement simple.
- Le dispositif d'injection d'implant comporte un boîtier, le pignon étant monté pivotant sur le boîtier. Ainsi, le mécanisme de transmission à crémaillère est réalisé de manière particulièrement simple.
- Le nombre de dents de la première denture est strictement inférieur au nombre de dents de la deuxième denture. Ainsi, la démultiplication du mouvement de l'utilisateur est réalisée de manière particulièrement simple. En outre, par exemple, le nombre de dents de la première denture est compris entre 5 et 15, de préférence entre 5 et 10, plus préférentiellement est voisin de ou égal à 5 ; et/ou le nombre de dents de la deuxième denture est compris entre 6 et 30, de préférence entre 16 et 25, plus préférentiellement voisin de ou égal à 18.
- Le rapport du déplacement du deuxième élément de crémaillère par rapport au déplacement du premier élément de crémaillère est compris entre 2 et 10, de préférence entre 3 et 5, plus préférentiellement est voisin de ou égal à 3,6. Ainsi, la démultiplication du mouvement de l'utilisateur est réalisée de manière optimale. En effet, si le rapport du déplacement du deuxième élément de crémaillère par rapport au déplacement du premier élément de crémaillère est trop important, la précision d'injection s'en trouve affectée, et si le rapport du déplacement du deuxième élément de crémaillère par rapport au déplacement du premier élément de crémaillère est trop faible, l'amplitude du mouvement à réaliser pour l'actionnement par l'utilisateur est trop importante.
- Le dispositif d'injection d'implant comporte des moyens de verrouillage agencés pour bloquer la tige de poussée dans sa position finale, position dans laquelle la tige de poussée fait de préférence saillie vers la direction aval au-delà de l'extrémité de l'aiguille d'injection. Ainsi, le dispositif d'injection d'implant n'est pas réutilisable, permettant de respecter les contraintes d'hygiènes relatives à un tel dispositif d'injection d'implant. En outre, lorsque dans sa position finale, la tige de poussée fait saillie vers la direction aval au-delà de l'extrémité de l'aiguille d'injection, la tige de poussée forme dans ce cas un élément de sécurité pour l'extrémité de l'aiguille d'injection, laquelle peut être biseautée pour faciliter le piquage. La tige de poussée permet alors d'empêcher l'aiguille d'exercer sa fonction de piquage, par exemple sur une personne ou un objet en cas de chute du dispositif d'injection d'implant. En effet, dans le cas d'une chute ou d'un appui dans la direction distale, le contact avec le dispositif d'injection d'implant est ainsi réalisé par l'intermédiaire de la tige de poussée et non avec l'extrémité de l'aiguille d'injection.

L'invention sera mieux comprise à la lecture de la description qui va suivre, donnée uniquement à titre d'exemple et faite en se référant aux dessins dans lesquels :
- la figure 1 est une vue en perspective d'un dispositif d'injection d'implant selon un mode de réalisation, en configuration de stockage avant injection ;
- les figures 2 et 3 sont des vues de côté et en perspective d'une partie du dispositif d'injection d'implant de la figure 1, dans lequel la tige de poussée est dans une position initiale ;
- la figure 4 est une vue de côté et en perspective d'une partie du dispositif d'injection d'implant de la figure 1, dans lequel la tige de poussée est dans une position intermédiaire ;
- les figures 5 à 7 sont des vues de côté et en perspective d'une partie du dispositif d'injection d'implant de la figure 1, dans lequel la tige de poussée est dans une position finale.

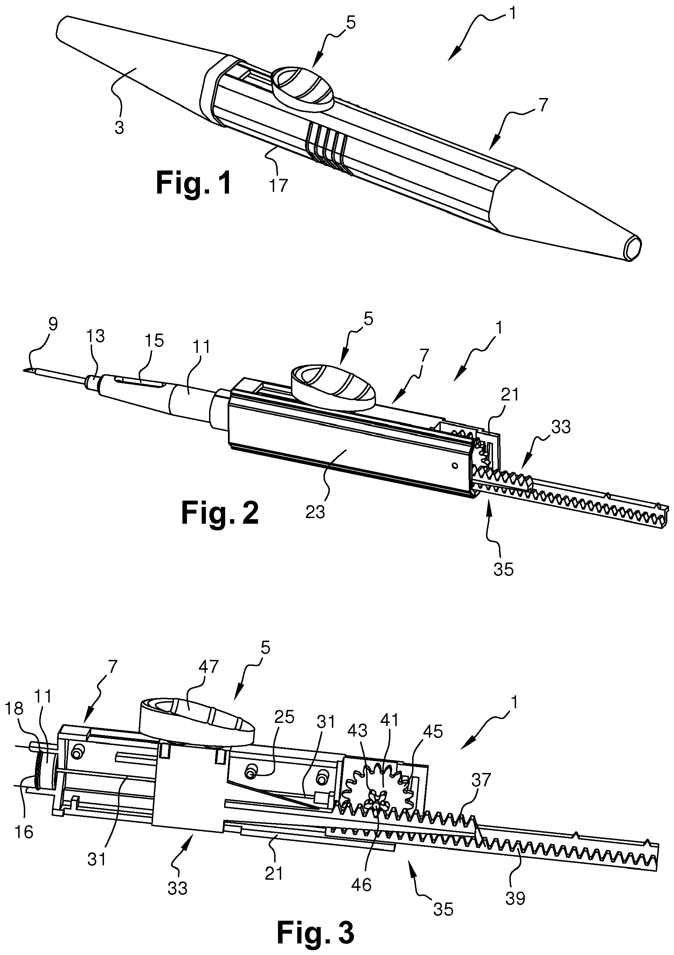

Comme cela est représenté sur les figures 1 et 2, un dispositif d'injection d'implant 1, comporte une aiguille d'injection 9 (visible sur la figure 2) portée par un boîtier de préhension 7 et protégée par un capuchon 3 (visible sur la figure 1), un corps de réception 11 (visible sur la figure 2), d'au moins un implant, des moyens d'injection 5 et un boîtier de préhension 7.

Le dispositif d'injection d'implant 1 est configuré pour injecter un ou plusieurs implants dans le corps d'un patient via l'aiguille d'injection 9 visible. Bien que dans ce qui suit, l'exemple soit illustré avec deux implants, le dispositif d'injection d'implant 1 est applicable également à un unique implant, ou un nombre d'implants supérieur à deux, comme par exemple trois, quatre, cinq, dix implants.

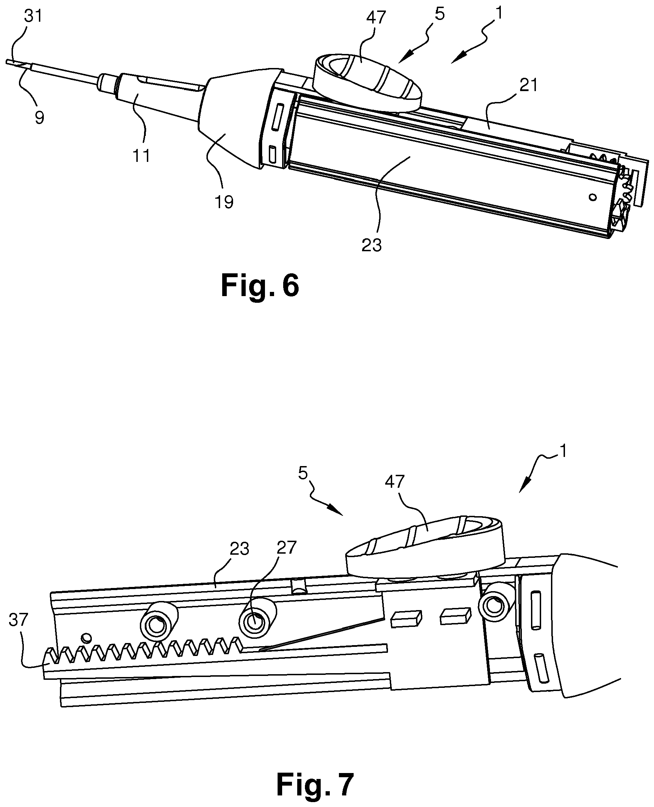

Comme cela est représenté sur les figures 2 et 6, l'aiguille d'injection 9 est creuse et est par exemple réalisée en métal comme de l'acier inoxydable. L'aiguille d'injection 9 comporte une extrémité distale biseautée pour faciliter son insertion dans le corps du patient. L'aiguille d'injection 9 porte un élément support 13, lequel peut être réalisé en matériau plastique et est destiné à limiter la profondeur d'insertion de l'aiguille d'injection 9 dans le corps du patient. L'aiguille d'injection 9 est rapportée à son extrémité proximale sur le corps de réception 11. Elle peut être protégée en configuration de stockage par le capuchon 3.

Le capuchon 3 est un capuchon de protection de l'aiguille d'injection 9, il est ici assemblé sur le boîtier de préhension 7, par encliquetage de son extrémité proximale sur le boîtier de préhension 7. Cependant, d'autres moyens d'assemblage sont possibles, par exemple par vissage. Le capuchon 3 est dans cet exemple en forme d'ogive, pouvant être muni de reliefs de manière à faciliter sa préhension.

Le corps de réception 11 est un corps de réception de deux implants. Comme illustré sur la figure 2, le corps de réception 11 est de forme générale tubulaire et/ou tronconique, et loge les implants dans son espace interne, de telle sorte que les implants soient orientés en direction de l'extrémité de l'aiguille d'injection 9, face à l'extrémité proximale de l'aiguille d'injection 9. En d'autres termes, le corps de réception 11 est disposé en amont de l'aiguille d'injection 9, et est destiné à recevoir les implants de sorte que les implants soient disposés en amont de l'aiguille d'injection 9, dans la direction d'injection de l'aiguille d'injection 9. Le corps de réception 11 est configuré pour contenir deux implants, les deux implants étant disposés l'un derrière l'autre, c'est-à-dire l'un en amont de l'autre, selon la direction d'injection. Le corps de réception 11 peut comporter, à son extrémité distale, un moyen de retenue d'implant, comme une membrane ou un faible rétrécissement de son diamètre interne, ayant pour but d'éviter la chute d'un implant à travers l'aiguille d'injection 9, par exemple sous l'action de la force de gravité terrestre. Le corps de réception 11 peut alternativement comporter, à son extrémité distale, un élément souple de retenue d'implant comportant un orifice d'un diamètre inférieur à celui des implants, lequel est configuré pour se déformer et permettre le passage de des implants vers l'aiguille d'injection 9 lors de l'injection. Le corps de réception 11 comporte avantageusement une fenêtre 15 (visible sur la figure 2). Ainsi, un utilisateur est en mesure de détecter visuellement, par la fenêtre 15, la présence des implants dans le dispositif d'injection d'implant 1 avant de réaliser l'injection sur un patient, après le retrait du capuchon 3. On notera que dans cet exemple, l'aiguille d'injection 9 et le corps de réception 11 sont des pièces rapportées l'une par rapport à l'autre, néanmoins il serait envisageable que l'aiguille d'injection 9 et le corps de réception 11 forment deux parties d'une même pièce, par exemple en étant venus de matière. Par ailleurs, le corps de réception 11 est rapporté à son extrémité proximale sur le boîtier de préhension 7, par exemple par encliquetage de son extrémité proximale sur le boîtier de préhension 7, comme cela est illustré sur la figure 3. Le corps de réception 11 comporte ainsi une nervure périphérique 16, laquelle s'engage dans une rainure 18 correspondante portée par le boîtier de préhension 7. Cependant, d'autres moyens d'assemblage sont possibles, par exemple par vissage. Selon une variante, le corps de réception 11 pourrait être réalisé directement dans le boîtier de préhension 7, en étant venu de matière avec ce dernier. Dans cet exemple, le corps de réception 11 est réalisé en un matériau plastique, éventuellement transparent.

Dans l'exemple illustré, le boîtier de préhension 7 se compose de plusieurs éléments :
- une partie externe, composée d'un élément externe de préhension 17 (visible sur la figure 1), de forme générale tubulaire, et d'un bouchon distal 19 (visible sur la figure 6), et
- une partie interne, composée de deux éléments de support 21, 23 des moyens d'injection 5 assemblés entre eux, à savoir un élément de support mâle 21 et un élément de support femelle 23 (visibles sur la figure 2).

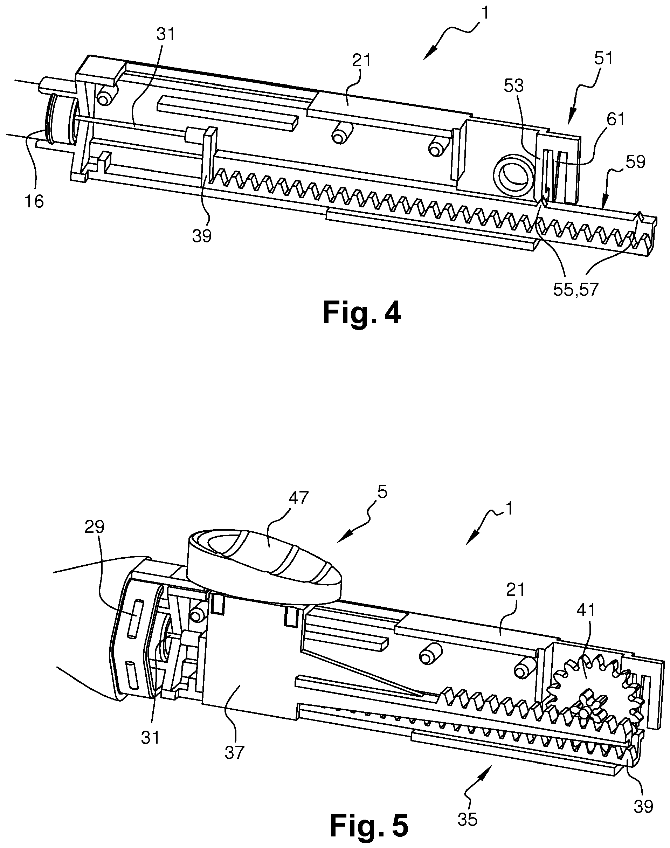

A l'état assemblé, l'élément externe de préhension 17, le bouchon distal 19 et les deux éléments de support 21, 23 sont maintenus en position les uns par rapport aux autres. Ainsi, les deux éléments de support 21, 23 sont assemblés par encliquetage de pions 25 (visibles sur la figure 3) portés par l'élément de support mâle 21 dans des logements tubulaires 27 (visibles sur la figure 7) portés par l'élément de support femelle 23. Par ailleurs à l'état assemblé, le bouchon distal 19 se trouve en butée contre l'extrémité distale des deux éléments de support 21, 23, et est assemblé à l'élément externe de préhension 17 par encliquetage de saillies 29, par exemple sous forme de tronçons hémicylindriques comme illustré sur la figure 5, dans une rainure périphérique interne correspondante de l'élément externe de préhension 17.

Les moyens d'injection 5 permettent de pousser le ou les implants au travers de l'aiguille d'injection 9 entre une position initiale et une position finale dans laquelle le ou les implants sont injectés. Comme on peut le voir notamment sur la figure 3, les moyens d'injection 5 comprennent une tige de poussée 31 et des moyens d'actionnement 33 par un utilisateur.

La tige de poussée 31 est disposée en amont des implants logés dans le corps de réception. La tige de poussée 31 s'étend longitudinalement et est configurée pour pousser les implants au travers de l'aiguille d'injection 9 entre une position initiale et une position finale dans laquelle les implants sont injectés.

La tige de poussée 31 peut être réalisée en métal, par exemple en acier, de préférence en acier inoxydable La tige de poussée 31 est disposée en amont d'un implant qui est lui-même l'implant disposé le plus en amont parmi les implants. La tige de poussée 31 est ainsi configurée pour pousser l'implant, ici les implants, au travers de l'aiguille d'injection 9 entre une position initiale et une position finale. Ainsi, dans la position initiale de la tige de poussée 31, les implants sont logés dans le corps de réception 11, et dans la position finale de la tige de poussée 31, les implants sont passés à travers l'aiguille d'injection 9 et se trouvent a priori placés dans le corps d'un patient.

Les moyens d'actionnement 33 par un utilisateur sont configurés pour actionner un déplacement de la tige de poussée 31 de la position initiale vers la position finale. Les moyens d'actionnement 33 comprennent un mécanisme de transmission à crémaillère 35.

Comme on peut le voir sur la figure 3, le mécanisme de transmission à crémaillère 35 comporte un premier élément de crémaillère 37 et un deuxième élément de crémaillère 39.

Le premier élément de crémaillère 37 est un élément de crémaillère entraînant. Il est ainsi configuré pour être entraîné par l'utilisateur et forme une première liaison de type pignon-crémaillère avec un pignon 41.

Comme illustré sur la figure 3, le pignon 41 comprend une première denture 43 coopérant avec le premier élément de crémaillère 37, et le pignon 41 comprend une deuxième denture 45 coopérant avec le deuxième élément de crémaillère 39 configuré pour pousser la tige de poussée 31 de la position initiale vers la position finale. Le pignon 41 forme ainsi une deuxième liaison de type pignon-crémaillère avec le deuxième élément de crémaillère 39. La première denture 43 et la deuxième denture 45 du pignon 41 sont réalisées respectivement par une première roue dentée 43 et une deuxième roue dentée 45, la première roue dentée 43 ayant un diamètre inférieur au diamètre de la deuxième roue dentée 45.

Le nombre de dents de la première denture 43 est strictement inférieur au nombre de dents de la deuxième denture 45, afin de réaliser une démultiplication du mouvement. Par exemple, le nombre de dents de la première denture 43 est compris entre 5 et 15, de préférence entre 5 et 10, plus préférentiellement est voisin de ou égal à 5, et/ou le nombre de dents de la deuxième denture 45 est compris entre 6 et 30, de préférence entre 16 et 25, plus préférentiellement voisin de ou égal à 18. Le nombre de dents de la première denture 43 et le nombre de dents de la deuxième denture 45 sont de préférence choisis pour que le rapport du déplacement du deuxième élément de crémaillère 39 par rapport au déplacement du premier élément de crémaillère 37 soit compris entre 2 et 10, de préférence entre 3 et 5, plus préférentiellement voisin de ou égal à 3,6.

Le pignon 41 est monté pivotant dans le boîtier de préhension 7. Le pignon 41 comporte pour cela un tourillon à chacune de ses extrémités, un tourillon étant porté par l'élément de support mâle 21, et un tourillon 46 étant porté par l'élément de support femelle 23. L'axe de rotation du pignon 41 est sensiblement orthogonal à la direction longitudinale de la tige de poussée 31, afin que le mouvement du deuxième élément de crémaillère 39 permette de pousser la tige de poussée 31 de la position initiale vers la position finale.

Le premier élément de crémaillère 37 supporte en outre un bouton d'actionnement 47, lequel est configuré pour être entraîné directement par l'utilisateur. Le bouton d'actionnement 47 est assemblé sur le premier élément de crémaillère 37, par exemple par encliquetage. On comprend que le premier élément de crémaillère 37 est d'un seul tenant avec le bouton d'actionnement 47.

Le bouton d'actionnement 47 est monté coulissant dans une direction sensiblement parallèle à la direction longitudinale de la tige de poussée 31. Pour cela, le premier élément de crémaillère 37 et l'ensemble formé par l'élément de support mâle 21 et l'élément de support femelle 23 forment une liaison glissière entre eux, dans la direction longitudinale de la tige de poussée 31.

Le deuxième élément de crémaillère 39 est un élément de crémaillère entraîné. Il est ainsi configuré pour être entraîné par le pignon 41 et forme une deuxième liaison de type pignon-crémaillère avec le pignon 41. Le deuxième élément de crémaillère 39 supporte la tige de poussée 31, la tige de poussée 31 étant par exemple fixée par serrage dans un logement du deuxième élément de crémaillère 39. Ainsi, lorsque le deuxième élément de crémaillère 39 est entraîné, il pousse la tige de poussée 31 dans la direction d'injection, depuis la position initiale vers la position finale.

Le deuxième élément de crémaillère 39 est monté coulissant dans une direction sensiblement parallèle à la direction longitudinale de la tige de poussée 31. Pour cela, le second élément de crémaillère 39 et l'ensemble formé par l'élément de support mâle 21 et l'élément de support femelle 23 forme une liaison glissière entre eux, dans la direction longitudinale de la tige de poussée. Ainsi, lorsqu'il est entraîné par le pignon 41, le deuxième élément de crémaillère 39 coulisse dans la direction longitudinale de la tige de poussée, provoquant le déplacement de la tige de poussée 31 dans la direction distale. La tige de poussée 31 pousse alors au moins un implant au travers de l'aiguille d'injection 9. Lorsque la tige de poussée 31 se trouve dans sa position finale, le deuxième élément de crémaillère 39 ne continue pas sa course. Le boîtier de préhension 7 forme alors, en particulier par le biais de l'élément de support mâle 21, une butée pour le deuxième élément de crémaillère 39. Ainsi, l'utilisateur ne peut injecter un implant sur une trop grande longueur susceptible de blesser le patient.

Le dispositif d'injection d'implant 1 comporte également des moyens d'indication 51 d'une position de la tige de poussée 31 à l'utilisateur, comme illustré par exemple sur la figure 4.

Les moyens d'indication 51 comprennent ici une première languette flexible 53 portée par le boîtier de préhension 7, en particulier par l'élément de support mâle 21. Le mécanisme de transmission à crémaillère 35 comprend au moins une saillie 55, de telle manière que lorsque le mécanisme de transmission à crémaillère 35 atteint une première position prédéterminée, la saillie 55 se trouve en butée contre la première languette flexible 53 de façon à fournir à l'utilisateur une première indication sonore et/ou tactile. Dans l'exemple illustré, la tige de poussée 31, dans cette première position prédéterminée du mécanisme de transmission par crémaillère 35, occupe une position dans laquelle un implant est injecté.

Dans l'exemple illustré, notamment sur la figure 4, la première languette flexible 53 est supportée par l'élément de support mâle 21 du boîtier de préhension 7. Le deuxième élément de crémaillère 39 supporte deux saillies 55, lesquelles comprennent chacune une dent 57 protubérante d'une surface longitudinale 59 plane du mécanisme de transmission à crémaillère 35. Par « surface longitudinale », on peut comprendre une surface s'étendant sensiblement parallèlement à la direction longitudinale de la tige de poussée 31. Dans l'exemple illustré, la surface longitudinale 59 plane est portée par le deuxième élément de crémaillère 39.

Lorsque le mécanisme de transmission à crémaillère 35 atteint une première position prédéterminée, par exemple une position dans laquelle un implant est injecté, voire une position intermédiaire dans laquelle un premier implant est injecté et un second implant n'est pas injecté et se trouve encore dans le dispositif d'injection d'implant 1, comme illustré sur la figure 4, la saillie 55 ou dent 57 se trouve en butée contre la première languette flexible 53. Dans le cas d'une position intermédiaire, afin d'injecter le second implant, l'utilisateur doit alors exercer un effort afin de provoquer la déformation de la première languette flexible 53 et permettre le franchissement par la première languette flexible 53 d'une saillie 55 ou dent 57.

Les moyens d'indication 51 comprennent une seconde languette flexible 61, portée par le boîtier de préhension 7, en particulier par l'élément de support mâle 21.

La seconde languette flexible 61 est configurée pour venir en butée contre une saillie 55 ou dent 57 dans une seconde position prédéterminée précédant la première position prédéterminée.

Lorsque le mécanisme de transmission à crémaillère 35 atteint une seconde position prédéterminée, par exemple une position dans laquelle un implant est partiellement injecté, voire une position dans laquelle un implant est proche d'être injecté en totalité, la saillie 55 ou dent 57 se trouve en butée contre la seconde languette flexible 61, et lorsque le mécanisme de transmission à crémaillère 35 dépasse la seconde position prédéterminée, la seconde languette flexible 61 franchit la saillie 55 ou dent 57 de façon à fournir à l'utilisateur une seconde indication sonore et/ou tactile. Dans cette seconde position prédéterminée, afin de continuer l'injection d'un implant, l'utilisateur doit alors exercer un effort afin de provoquer la déformation de la seconde languette flexible 61 et permettre le franchissement par la seconde languette flexible 61 d'une saillie 55 ou dent 57.

La première languette flexible 53 comporte une résistance à la flexion plus élevée que la seconde languette flexible 61. Ainsi, l'utilisateur distingue facilement la première position prédéterminée de la seconde position prédéterminée. Lors de l'injection d'un implant, la seconde position prédéterminée va tout d'abord être atteinte, indiquant par exemple à l'utilisateur l'approche de l'injection en totalité de l'implant. Puis, la première position prédéterminée est atteinte, indiquant par exemple à l'utilisateur l'injection en totalité de l'implant. Cela peut par exemple permettre à l'utilisateur de repositionner le dispositif d'injection d'implant 1, en particulier l'aiguille d'injection 9, de manière à pouvoir débuter l'injection d'un autre implant avec un positionnement adéquat.

Dans l'exemple illustré, le dispositif d'injection d'implant 1 comprenant deux saillies 55 ou dent 57, le mécanisme de transmission à crémaillère 35 peut ainsi occuper deux premières positions prédéterminées, respectivement deux secondes positions prédéterminées. En particulier, une première position prédéterminée et une seconde position prédéterminée sont atteintes lors de l'injection de chacun des deux implants.

Le dispositif d'injection d'implant 1 comporte des moyens de verrouillage agencés pour bloquer la tige de poussée 31 dans sa position finale, position dans laquelle la tige de poussée 31 fait de préférence saillie vers la direction aval au-delà de l'extrémité de l'aiguille d'injection 9.

De plus, le dispositif d'injection d'implant 1 peut comporter des moyens de verrouillage agencés pour bloquer la tige de poussée 31 dans la position finale. Ainsi dans cette position finale, un ergot supporté par le premier élément de crémaillère 37 ou le second élément de crémaillère 39 coopère avec un évidement porté par le boîtier de préhension 7, en particulier ménagé dans l'élément de support mâle 21. Avantageusement, l'ergot peut être ménagé sur la surface longitudinale 59 du deuxième élément de crémaillère 39 et se présenter sous la forme d'une rampe se terminant à son extrémité amont par une paroi sensiblement orthogonale à la surface longitudinale, afin que le franchissement de cette rampe par la première languette flexible 53 ou la seconde languette flexible 61 génère un signal sonore audible par l'utilisateur, comme un 'clic'. De plus, la présence de la paroi sensiblement orthogonale ne permet pas le franchissement par l'ergot dans l'autre sens, sauf par exemple à provoquer la rupture de la première languette flexible 53 ou de la seconde languette flexible 61. Cela évite de manière simple la réutilisation du dispositif d'injection d'implant 1 et permet en outre d'éviter une blessure due à l'aiguille d'injection 9, par exemple en cas de chute du dispositif d'injection d'implant 1 après utilisation, grâce au fait que la tige de poussée 31 fait saillie au-delà de l'extrémité de l'aiguille d'injection 9 et est bloquée par ces moyens de verrouillage.

Les éléments du dispositif d'injection d'implant 1 dont le matériau n'est pas précisé dans la présente description peuvent être réalisés en matériau thermoplastique, par exemple en polyéthylène ou en polypropylène.

Un exemple de fonctionnement du dispositif d'injection d'implant 1 va à présent être décrit.

Le dispositif d'injection d'implant 1 comme illustré sur la figure 1 se trouve en configuration de stockage, avant utilisation.

L'utilisateur doit retirer le capuchon 3 de protection de l'aiguille d'injection 9, comme illustré sur la figure 2 - le dispositif d'injection d'implant 1 étant considéré assemblé - il vérifie la présence de l'implant ou des implants par visualisation à travers la fenêtre 15 du corps de réception 11.

L'aiguille d'injection 9 est ensuite enfoncée dans le corps du patient, et l'utilisateur fait coulisser sur le bouton d'actionnement 47. Le bouton d'actionnement 47 et le premier élément de crémaillère 37 coulissent alors relativement au boîtier de préhension 7. Le premier élément de crémaillère 37 entraîne alors le pignon 41 en rotation par l'intermédiaire de sa première denture 43. La rotation du pignon 41, par l'intermédiaire de sa deuxième denture 45 en contact avec le deuxième élément de crémaillère 39, provoque le coulissement du deuxième élément de crémaillère 39. Le deuxième élément de crémaillère 39 pousse alors la tige de poussée 31 dans la direction d'injection, depuis sa position initiale vers sa position finale. La tige de poussée 31 pousse alors le ou les implants au travers de l'aiguille d'injection 9, de manière provoquer l'injection du ou des implants dans le corps du patient.

Ensuite, lorsque le mécanisme de transmission à crémaillère 35 atteint une seconde position prédéterminée, par exemple une position dans laquelle une injection d'un implant est proche d'être achevée, la saillie 55 ou dent 57 se trouve en butée contre la seconde languette flexible 61. L'utilisateur, s'il continue à appuyer sur le bouton d'actionnement 47, doit alors exercer un effort pour que le mécanisme de transmission par crémaillère 35 dépasse la seconde position prédéterminée. Cela provoque une déformation de la seconde languette flexible 61 et permet à la seconde languette flexible 61 de franchir la saillie 55 ou dent 57 de façon à fournir à l'utilisateur une seconde indication sonore, comme un 'clic', et/ou tactile, due par exemple à l'effort supplémentaire à fournir pour franchir la saillie 55 ou dent 57.

Après cela, lorsque le mécanisme de transmission atteint une première position prédéterminée, par exemple une position dans laquelle la tige de poussée 31 occupe une position dans laquelle un implant est injecté comme illustré sur la figure 4, la saillie 55 ou dent 57 se trouve en butée contre la première languette flexible 43. L'utilisateur, s'il continue à appuyer sur le bouton d'actionnement 47, doit alors exercer un effort pour que le mécanisme de transmission par crémaillère 35 dépasse la première position prédéterminée. Cela provoque une déformation de la première languette flexible 53 et permet à la première languette flexible 53 de franchir la saillie 55 ou dent 57 de façon à fournir à l'utilisateur une première indication sonore, comme un 'clic', et/ou tactile, due par exemple à l'effort supplémentaire à fournir pour franchir la saillie 55 ou dent 57.

Par exemple, la première languette flexible 53 comporte une résistance à le flexion plus élevée que la seconde languette flexible 61, de manière à ce que l'utilisateur distingue facilement si l'injection d'un implant est proche d'être achevée ou bien si cet implant est injecté.

Dans l'exemple illustré, le deuxième élément de crémaillère 39 comprend deux dents 57, positionnées l'une derrière l'autre de manière à correspondre à l'injection d'un premier implant puis d'un second implant. Ainsi, la première dent 57, positionnée en aval par rapport à la seconde dent 57, coopère avec la seconde languette 61 puis la première languette 53 lors de l'injection d'un premier implant, et la seconde dent 57 coopère avec la seconde languette 61 puis la première languette 53 lors de l'injection d'un second implant.

Enfin, lorsque la tige de poussée 31 atteint sa position finale, comme illustré sur les figures 5 et 6, celle-ci fait saillie vers la direction aval au-delà de l'extrémité de l'aiguille d'injection 9. Dans cette position, l'élément de support mâle 21 forme une butée pour le deuxième élément de crémaillère 39. Dans cette position, les implants sont tous injectés.

L'invention n'est pas limitée aux modes de réalisation présentés et d'autres modes de réalisation apparaîtront clairement à l'homme du métier. Bien que l'invention ait été illustrée avec un dispositif d'injection d'implant 1 configuré pour injecter deux implants, l'homme du métier comprendra aisément qu'un tel dispositif d'injection d'implant 1 peut être configuré pour injecter un unique implant, par exemple doté d'une grande longueur, ou bien un nombre d'implants supérieur à deux.

## Revendications

1. Dispositif d'injection d'implant (1), **caractérisé en ce qu'**il comporte :
- une aiguille d'injection (9),
- un corps de réception (11) d'au moins un implant,
- des moyens d'injection (5), les moyens d'injection (5) comprenant :
. une tige de poussée (31), disposée en amont de l'implant logé dans le corps de réception (11), s'étendant longitudinalement et configurée pour pousser l'implant au travers de l'aiguille d'injection (9) entre une position initiale et une position finale dans laquelle l'implant est injecté,
. des moyens d'actionnement (33) par un utilisateur, configurés pour actionner un déplacement de la tige de poussée (31) de la position initiale vers la position finale,
les moyens d'actionnement (33) comprenant un mécanisme de transmission à crémaillère (35),
- des moyens de verrouillage agencés pour bloquer la tige de poussée (31) dans sa position finale, position dans laquelle la tige de poussée (31) fait de préférence saillie vers la direction aval au-delà de l'extrémité de l'aiguille d'injection (9).

2. Dispositif d'injection d'implant (1) selon la revendication précédente, dans lequel le mécanisme de transmission à crémaillère (35) comporte un premier élément de crémaillère (37) et un deuxième élément de crémaillère (39),
. le premier élément de crémaillère (37) étant configuré pour être entraîné par l'utilisateur et formant une première liaison de type pignon-crémaillère avec un pignon (41), le pignon (41) comprenant une première denture (43) coopérant avec le premier élément de crémaillère (37),
. le pignon (41) comprenant une deuxième denture (45) coopérant avec le deuxième élément de crémaillère (39) configuré pour pousser la tige de poussée (31) de la position initiale vers la position finale.

3. Dispositif d'injection d'implant (1) selon la revendication précédente, dans lequel les moyens d'actionnement (33) comportent un bouton d'actionnement (47) coulissant dans une direction sensiblement parallèle à la direction longitudinale de la tige de poussée (31), lequel est supporté par le premier élément de crémaillère (37).

4. Dispositif d'injection d'implant (1) selon l'une quelconque des revendications précédentes, comportant des moyens d'indication (51) d'une position de la tige de poussée (31) à l'utilisateur.

5. Dispositif d'injection d'implant (1) selon la revendication précédente, dans lequel les moyens d'indication (51) comprennent une première languette flexible (53) portée par un boîtier (7, 21), et le mécanisme de transmission à crémaillère (35) comprenant au moins une saillie (55), de telle manière que lorsque le mécanisme de transmission à crémaillère (35) atteint une première position prédéterminée, la saillie (55) se trouve en butée contre la première languette flexible (53) de façon à fournir à l'utilisateur une première indication sonore et/ou tactile.

6. Dispositif d'injection d'implant (1) selon la revendication précédente, dans lequel les moyens d'indication (51) comportent une seconde languette flexible (61) portée par le boîtier (7, 21), la seconde languette flexible (61) étant configurée pour venir en butée contre la saillie (55) dans une seconde position prédéterminée précédant la première position prédéterminée, de telle manière que lorsque le mécanisme de transmission à crémaillère (35) atteint une seconde position prédéterminée, la saillie (55) se trouve en butée contre la seconde languette flexible (61), et lorsque le mécanisme de transmission à crémaillère (35) dépasse la seconde position prédéterminée, la seconde languette flexible (61) franchit la saillie de façon à fournir à l'utilisateur une seconde indication sonore et/ou tactile.

7. Dispositif d'injection d'implant (1) selon la revendication précédente, dans lequel la première languette flexible (53) comporte une résistance à la flexion plus élevée que la seconde languette flexible (61).

8. Dispositif d'injection d'implant (1) selon l'une quelconque des revendications 5 à 7, dans lequel
le corps de réception (11) est apte à recevoir une pluralité d'implants,
la tige de poussée (31), dans la première position prédéterminée du mécanisme de transmission à crémaillère (35), occupe une position dans laquelle un implant est injecté.

9. Dispositif d'injection d'implant selon l'une quelconque des revendications 5 à 8, dans lequel la au moins une saillie (55) comprend une dent (57) protubérante d'une surface longitudinale (59) plane du mécanisme de transmission à crémaillère (35).

10. Dispositif d'injection d'implant (1) selon l'une quelconque des revendications 2 à 9, comportant un boîtier (7, 21, 23), le pignon (41) étant monté pivotant sur le boîtier (7, 21, 23).

11. Dispositif d'injection d'implant (1) selon l'une quelconque des revendications 2 à 10, dans lequel le nombre de dents de la première denture (43) est strictement inférieur au nombre de dents de la deuxième denture (45).
